# EUROPEAN PATENT APPLICATION

(11) **EP 2 407 768 A1**
(43) Date of publication of application: **18.01.2012**
(21) Application number: 10750581.0
(22) Date of filing: 10.03.2010
(51) Int. Cl.: G01N 15/06, G01N 3/00

(54) **HARD PARTICLE CONCENTRATION DETECTION METHOD, PARTICLE CONCENTRATION DETECTION METHOD, AND DEVICE THEREFOR**

(30) Priority: 12.03.2009 JP 2009059469; 24.12.2009 JP 2009291799
(71) Applicant: IHI Corporation, Tokyo 135-8710 (JP); Diesel United, Ltd., Tokyo 101-0041 (JP)
(72) Inventor: UKAI, Hidemi, Tokyo 135-8710 (JP); FUJII, Takashi, Aioi-shi Hyogo 678-0041 (JP)
(74) Representative: Köhler, Walter
(86) International application number: PCT/JP2010/001697
(87) International publication number: WO 2010/103824

(57) **Abstract**

Magnetic and counterpart members 12 and 11 are immersed in a liquid which may include hard particles. At least one of the members 12 and 11 is moved while pressed to the other member. The magnetic member 12 is worn by hard particles in the liquid to produce magnetic particles in the liquid. A concentration of the magnetic particles produced is measured and is converted into a concentration of hard particles in the liquid on the basis of a calibration line representing a correlation measured in advance between the concentrations of the magnetic particles and of the hard particles in the liquid. Thus, the concentration of the hard particles in the liquid is detected.

## Description

### Technical Field

The invention relates to a concentration detecting method for hard particles in a liquid, a concentration detecting method for particles in a liquid, and a device therefor.

### Background Art

Generally mixed in a liquid such as bunker C, which is a main fuel for a marine diesel engine, are alumina, silica, carbon or other hard particles as residue in fluid catalytic cracking (FCC) for petroleum refinery.

Excessive inflow of such particles into the engine with a piston ring, a cylinder liner and the like may cause adverse effects such as degraded sliding, seizing-up and mechanical wear. Thus, every time fuel is replenished, a ship management company samples and chemically analyzes the fuel to quantitatively grasp the particles in the fuel. When fuel with particles of not less than a stipulated concentration is replenished to a ship, the fact is notified of to the ship's crew to call their attention.

Conventionally, when particles in fuel are to be detected, sampled fuel is filtered through a filter or the like and a residue is microscopically observed or quantitatively analyzed to detect particles.

State-of-the-art technology with respect to a hard particle concentration detesting method, a particle concentration detecting method, and a device therefor is shown, for example, in Patent Literature 1.

### Citation List

### Patent Literature

[Patent Literature 1: JP 11-153541A

### Summary of Invention

### Technical Problems

However, any conventional particle concentration detecting method and a device therefor require a specific number of days until an analyzed result is reported to the ship's crew. Therefore, when the fuel requires to be used pending the analysis result, a problem arises that adverse effects on the driving engine cannot be preliminarily prevented. A large amount of particles may be suddenly supplied to the engine when a particle-including contaminant is precipitated to increase the concentration of the particles in the fuel during storage or when malfunction of a filter, a centrifugal separation cleaner or the like occurs in a fuel treatment system from a fuel tank to an inlet of the engine. Thus, continuous and quantitative grasp of the particles in the fuel is demanded.

There is also a problem that alumina, silica or other particles, which have no remarkable features in magnetism and in electric conductivity, are difficult to magnetically or electrically detect. There is further a problem that the particles, which are chemically stable, are difficult to detect utilizing a chemical reaction. There is still further a problem that the bunker C or the like liquid, which is opaque and highly viscous and includes various sludge or other particles in addition to the alumina and silica particles, cannot be sufficiently dealt with using optical determination as described in Patent Literature 1.

The invention was made in view of the above and has its object to provide a hard particle concentration detecting method which quickly and quantitatively grasps hard particles in a liquid, a particle concentration detecting method which continuously and quantitatively grasps particles in a liquid, and a device therefor.

### Solution to Problems

A hard particle concentration detecting method of the invention comprises the steps of immersing magnetic and counterpart members in a liquid which may include hard particles, moving at least one of the members while the member is pressed to the other member, wearing the magnetic member by hard particles in the liquid to produce magnetic particles in the sample liquid, measuring a concentration of the magnetic particles produced, and converting the measured concentration of the magnetic particles into a concentration of hard particles in the liquid on the basis of a calibration line representing a correlation measured in advance between the concentrations cf the magnetic particles and of the hard particles in the liquid to thereby detect the concentration of the hard particles in the liquid.

In the hard particle concentration detecting method of the invention, it is preferable that said at least one of magnetic and counterpart members is moved while pressed to the other member and with the hard particles in the liquid being between the members.

A particle concentration detecting method of the invention is that providing a magnetic particle producing part positioned in a flow path of a liquid which may include particles and having magnetic and counterpart members arranged therein as well as a magnetic particle measuring part positioned in the flow path same as that for the magnetic particle producing part to measure a concentration of magnetic particles in the liquid, said method comprising the steps of, when a concentration of particles is to be measured,
moving at least one of the members while the member is pressed to the other member, wearing the magnetic member to produce magnetic particles in the liquid, measuring a concentration of the produced magnetic particles by the magnetic particle measuring part, converting the measured concentration of the magnetic particles into a concentration of particles in the liquid on the basis of a calibration line representing a correlation measured in advance between the concentrations of the magnetic particles and of the particles in the liquid, thereby detecting the concentration of the particles in the liquid.

In the particle concentration detecting method of the invention, it is preferable that a concentration of magnetic particles originally included in the liquid is measured before the production of the magnetic particles by the magnetic particle producing part, said concentration of the magnetic particles originally included in the liquid being subtracted from the concentration of the magnetic particles produced in the liquid by the magnetic particle producing part, a subtracted result being converted into the concentration of the particles,

A particle concentration detecting device of the invention comprises
a magnetic particle producing part having magnetic and counterpart members arranged in a flow path of a liquid which may include particles for moving at least one of said members while the member is pressed to the other member and wearing the magnetic member to produce magnetic particles,
a magnetic particle measuring part positioned in the same flow path as that of the magnetic particle producing part for measuring the concentration of the magnetic particles in the liquid, and
a control part for converting the concentration of the magnetic particles measured by the magnetic particle measuring part into a concentration of the particles in the liquid on the basis of a calibration line representing a correlation measure in advance between concentrations of the magnetic particles and of the particles in the liquid, thereby detecting the concentration of the particles included in the liquid.

In the particle concentration detecting device of the invention, it is preferable that a preceding magnetic particle measuring part is arranged upstream of the magnetic particle producing part to measure a concentration of the magnetic particles originally included in the liquid.

In the particle concentration detecting device of the invention, it is preferable that the magnetic particle measuring part comprises a detector body connected to the flow path of the liquid, a movable partition for communication of the flow path with an inside of the detector body such that the liquid in the flow path may be introduced into the detector body, an exciting coil positioned external to the detector body, an output coil positioned external to the detector body for generating an exciting voltage by an AC current of the exciting coil and a signal processing unit for measuring a variation in phase difference between the exciting and output coils.

In the particle concentration detecting device of the invention, it is preferable that the detector body of the magnetic particle measuring part is arranged communicably with an inflow-side flow path to the magnetic particle producing part and with an outflow-side flow path from the magnetic particle producing part,
the movable partition of the magnetic particle measuring part comprising an inflow-side piston body arranged for the inflow-side flow path, an outflow-side piston body arranged for the outflow-side flow path, an intermediate piston body arranged between the inflow-and outflow-side piston bodies and a piston rod having the inflow- and outflow-side and intermediate piston bodies arranged thereon,
whereby movement of the piston rod in one direction brings about switching into a state where the inflowside flow path is in communication with the inside of the detector body due to the inflow-side and intermediate piston bodies and the liquid flowing through the inflow-side flow path is introduced into the detector body; and movement of the piston rod in the other direction brings about switching into a state where the outflow-side flow path is in communication with the inside of the detector body due to the outflow-side and intermediate piston bodies and the liquid flowing through the outflow-side flow path is introduced into the detector body.

In the particle concentration detecting device of the invention, it is preferable that the inflow-side flow path is provided with a temperature controlling unit for controlling a temperature on the inflow side and a flow adjusting unit for feeding the liquid at a constant flow rate.

### Advantageous Effects of Invention

According to the hard particle concentration detecting method of the invention, presence of the hard particles in the liquid is used to wear the magnetic material to thereby produce the magnetic particles in the liquid; and the concentration of the magnetic particles produced is measured, and is converted into the concentration of the hard particles in the liquid by use of the calibration line to thereby detect the concentration of the hard particles in the liquid. As a result, the hard particles in the liquid can be quickly and quantitatively grasped. When the liquid is an oil, the states can be prevented where untested fuel is used and where a large amount of hard particles are suddenly supplied to a driving engine, thereby suppressing any adverse effect on the driving engine. The fact that the concentration of the hard particles is indirectly detected using the magnetic particles produced by the wearing of the magnetic member brings about excellent effects that there is no necessity of any operation and process for directly detecting the hard particles through physical or chemical treatment of the liquid itself and that favorably the hard particles in the liquid can be quickly and quantitatively grasped.

According to the particle concentration detecting method and the device therefor of the invention, the magnetic material is worn to produce the magnetic particles; and the concentration of the magnetic particles produced is measured, and is converted into the concentration of the particles in the liquid by use of the calibration line to thereby detect the concentration of the particles in the liquid; as a result, the particles in the liquid can be quantitatively grasped. The magnetic particle producing and measuring parts are provided in the same flow path, so that the concentration of the particles in the liquid can be continuously grasped. When the liquid is a fuel oil, the states can be prevented where untested fuel is used and where a large amount of particles are suddenly supplied to a driving engine, thereby suppressing any adverse effect on the driving engine. The fact that the concentration of the particles is indirectly detected using the magnetic particles produced by the wearing of the magnetic member brings about excellent effects that there is no necessity of any operation and process for directly detecting the particles through physical or chemical treatment of the liquid itself and that favorably the particles in the liquid can be continuously and quantitatively grasped.

### Brief Description of Drawings

Fig. 1 is a flowchart of a process procedure of a hard particle concentration detecting method of the invention;
Fig. 2 is a general conceptual view showing a magnetic particle producing part in the hard particle concentration detecting method of the invention;
Fig. 3 is a conceptual view showing, in enlarged scale, magnetic and counterpart members in the magnetic particle producing part of Fig. 2;
Fig. 4 is a view looking in the direction of arrows IV in Fig. 3;
Fig. 5a is a conceptual view showing magnetic and counterpart members in an alternative magnetic particle producing part;
Fig. 5b is a conceptual view showing magnetic and counterpart members in a further alternative magnetic particle producing part;
Fig. 5c is a conceptual view showing magnetic and counterpart members in a still further alternative magnetic particle producing part;
Fig. 6 is a general conceptual view showing an example of a magnetic particle measuring part in the hard particle concentration detecting method of the invention;
Fig. 7 is a general conceptual view showing an alternative example of the magnetic particle measuring part in the hard particle concentration detecting method of the invention;
Fig. 8 is a block diagram showing a configuration of a signal processing unit in the magnetic particle measuring part;
Fig. 9 is a conceptual diagram of processes from an output signal to an output value for comparison under no influence of magnetic particles;
Fig. 10 is a conceptual diagram of processes from the output signal to an output value for a concentration of magnetic particles under the influence of the magnetic particles;
Fig. 11 is a graph showing a relationship between a driving time period (grinding time period) and a concentration of magnetic particles (magnetic powder Fe) in the magnetic particle producing part;
Fig. 12 shows a calibration line representing a relationship between concentrations of hard particles and of the magnetic particles (magnetic powder);
Fig. 13 is a conceptual view showing a position of a particle concentration detecting device of the invention;
Fig. 14 is a general conceptual view showing a configuration of the particle concentration detecting device of the invention with a piston thereof being moved downward;
Fig. 15 is a general conceptual view showing the configuration of the particle concentration detecting device of the invention with the piston thereof being moved upward;
Fig. 16a is a conceptual view showing an example of the magnetic particle producing part;
Fig. 16b is a conceptual view showing an alternative example of the magnetic particle producing part;
Fig. 16c is a conceptual view of a still alternative example of the magnetic particle producing part;
Fig. 17 is a block diagram showing a configuration of a signal processing unit in the magnetic particle measuring part;
Fig. 18 is a general conceptual view showing a further example of the configuration of the particle concentration detecting device of the invention;
Fig. 19 is a flowchart of fluid flow in a particle concentration detecting method of the invention; and
Fig. 20 is a graph showing a relationship between a time period and signals for concentrations of the magnetic particles when a movable partition of the magnetic particle measuring part is driven.

### Description of Embodiments

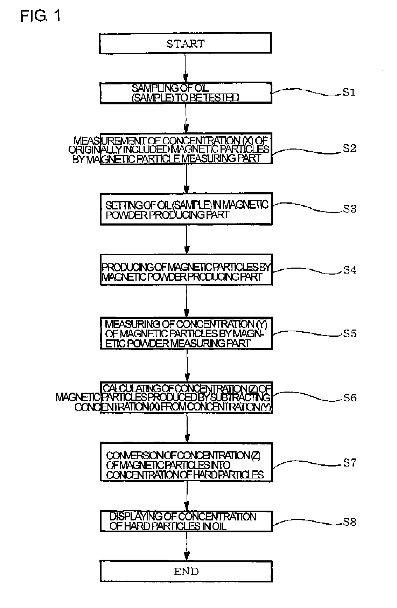

An embodiment of a hard particle concentration detecting method according to the invention will be described with reference to Figs. 1 to 12.

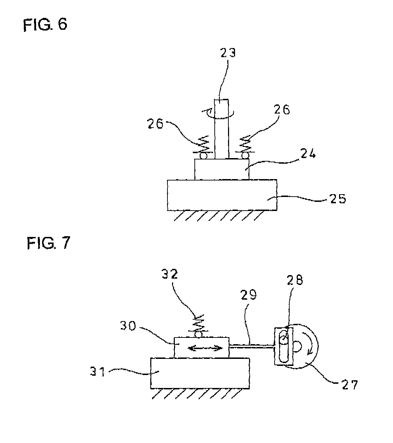

The embodiment provides a magnetic particle producing part (magnetic powder producing part) 1 for production of magnetic particles (magnetic powder) in a liquid, a magnetic particle measuring part 2 (see Figs. 6 to 8) for measurement of a concentration of the magnetic particles in the liquid and a control part 3 (see Figs. 6 and 7) for processing of the concentration of the magnetic particles. The embodiment will be described with respect to a case where the liquid is an oil.

The magnetic particle producing part 1 comprises a motor or other drive 5 with a rotating shaft 4 therebeneath, a holder 6 on the drive 5 to surround the shaft 4, a rod 7 connected to the shaft 4 to extend downward and outward of the holder 6, a sleeve 8 supported by the holder 6 and fitted over the rod 7, a spring or other resilient member 9 biasing the sleeve 8 downward and wear-plate counterpart and plate-like magnetic members 11 and 12 arranged underneath the sleeve 8 using a nut or other fixing member 10.

The drive 5 and the holder 6 are fixed to a pedestal (not shown) to expose the rod 7, the sleeve 8 and the like downward. The holder 6 has a downward protrusion 14 over which a test tube or other container 13 with a fuel or other oil S therein can be fitted. The protrusion 14 has a circumferential groove with an 0-ring 15 fitted therein.

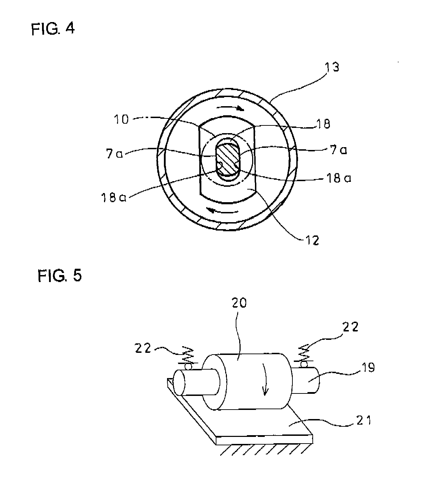

The rod 7 is of a length accommodable in the container 13 together with the members 8, 9, 11 and 12 when the container 13 is arranged on the holder 6, and is adapted to be rotated by the drive 5. The rod 7 has a lower portion circumferentially oppositely and planarly cut out to provide opposite side faces 7a (see Fig. 4).

The sleeve 8 is formed with an upper support 16 for support of a lower portion of the resilient member 9 as well as a lower support 17 abutting on the counterpart member 11. The resilient member 9 is supported by the protrusion 14 on the holder 6 and biases the counterpart member 11 downward through the sleeve 8 to thereby press the member 11 against the magnetic member 12. Preferably, the pressure to the members 11 and 12 is adjusted by exchanging the spring or other resilient member 9.

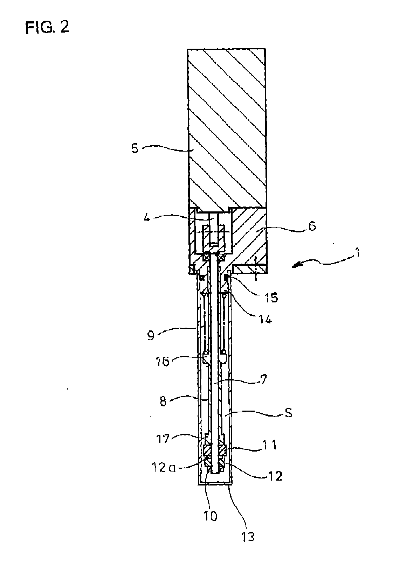

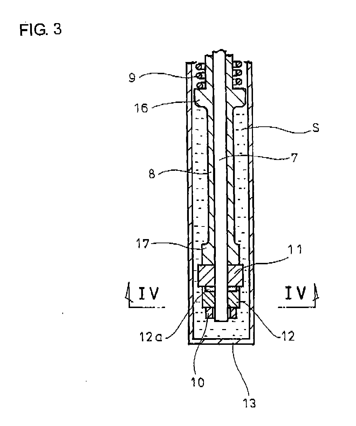

The counterpart member 11 has a bore (not shown) for passing of the rod 7 therethrough and is adapted so as not to follow the rotation of the rod 7 whereas the magnetic member 12 has a bore 18 (see Fig. 4) for passing of the rod 7 therethrough to provide opposite planar portions 18a on which the side faces 7a of the rod 7 lower portion can abut so that the magnetic member 12 follows the rotation of the rod 7. The magnetic member 12 is made of an iron-based or other material having magnetism. The counterpart member 11 is made of carbon steel or other material harder and less wearable than the magnetic member 12. Either of the members 11 and 12 (the member 12 in Figs. 2 and 3) is formed with a groove 12a which faces the other member. When the magnetic member 12 is rotated with hard particles being between the members 11 and 12, the magnetic member 12 is scraped off into abrasive wear by the hard particles. The material of the magnetic member 12 is not limited to iron and may be any, provided that magnetic particles (magnetic powder) with a required diameter may be produced due to the wearing. The material of the counterpart member 11 may be different from or the same as that of the magnetic member 12, provided that the magnetic particles may be produced from the magnetic member 12. The magnetic and counterpart members 12 and 11 may be arranged with their positions exchanged.

An alternative example of the magnetic particle producing part 1 may be employed which has components different from the members 12 and 11 and the like. More specifically, it comprises, as shown in Fig. 5a, a shaft 19 rotated by a drive (not shown), a roller 20 positioned centrally over the shaft 19, a plate 21 fixed to a stationary member (not shown) and abutting on the roller 20 and springs or other resilient members 22 biasing the shaft 19 to press the roller 20 onto the plate 21. Either of the roller 20 and the plate 21 is a magnetic member and the other is a counterpart member.

A further alternative example may be employed which comprises, as shown in Fig. 5b, a shaft 23 rotated by a drive (not shown), a rotating plate 24 positioned on a tip of the shaft 23, a plate 25 fixed to a stationary member (not shown) and abutting on a bottom of the rotating plate 24 and springs or other resilient members 26 pressing the rotating plate 24 against the plate 25. Either of the plates 24 and 25 is a magnetic member and the other is a counterpart member.

A still further alternative example may be employed which comprises, as shown in Fig. 5c, an eccentric pin 28 rotated by a drive 27, a connecting member 29 to convert a motion of the eccentric pin 28 into a reciprocating motion, a reciprocating plate 30 connected to the connecting member 29, a plate 31 fixed to a stationary member (not shown) and abutting on a bottom of the reciprocating plate 30 and a spring or other resilient member 32 to press the reciprocating plate 30 against the plate 31. Either of the plates 30 and 31 is a magnetic member and the other is a counterpart member.

Next, an example of the magnetic particle measuring part 2 will be described though it is not especially limited in construction, providing a concentration of the magnetic particles can be measured in ppm. As shown in Figs. 6 to 10, the exemplary magnetic particle measuring part 2 comprises a flow path L of an oil S which may include magnetic particles. The flow path L is provided with a fluid lead-out/lead-in guide unit 33 and a detecting unit 34. The detecting unit 34 is connected to a signal processing unit 35 which in turn is connected to a concentration measuring unit 36 for conversion of a signal from the signal processing unit 35 into a concentration of the magnetic particles.

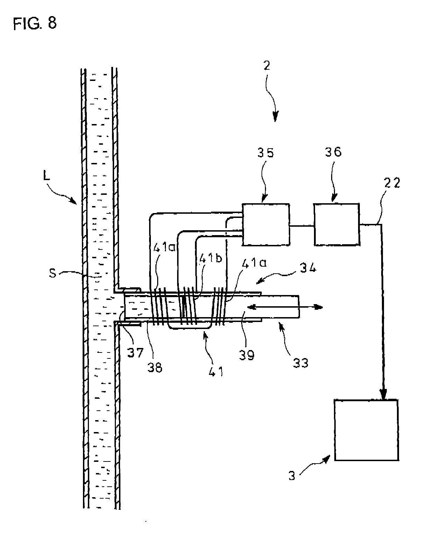

The lead-out/lead-in guide unit 33 comprises a cylindrical detector body 38 opened at 37 to the flow path L, a piston 39 which slides inside the detector body 38 for guided lead-out/lead-in of the oil S, a drive or rotating body 40 (see Fig. 8) for forward/backward movement of the piston 39 and coils 41 of the detecting unit 34 arranged on a circumference of the detector body 38. The flow path L may be a pipe, a tube or any other, provided that the oil S flows therethrough.

The coils 41 of the detecting unit 34 are two exciting coils 41a wound in opposite directions to each other and connected in series and a detection coil (output coil) 41b arranged between and adjacent to the coils 41a. The detection coil 41b is adapted to generate an output signal indicative of an AC voltage (exciting voltage) when the AC voltage is applied to the exciting coils 41a. The exciting and detection coils 41a and 41b are adjusted to have substantially uniform mutual inductance by adjusting wound number and distance of the coils 41. The exciting and detection coils 41a and 41b have no limitation in their numbers.

Alternatively, as shown in Fig. 7, the coils 41 of the detecting unit 34 may be an exciting coil 41c and a detection coil (output coil) 41d arranged adjacent thereto. Also in this case, the detection coil 41d is adapted to generate an output signal indicative of an AC voltage (exciting voltage) when the AC voltage is applied to the exciting coil 41c. It is adjusted such that the output signal indicative of the AC voltage (exciting voltage) in the detection coil 41d is small when no magnetic particles are detected.

As shown in Fig. 8, in order to acquire detection or corrective detection signal of the magnetic particles from the output signal of the detection coil 41b, the signal processing unit 35 comprises an amplifier circuit 42 connected to the coil 41b to amplify a feeble waveform signal, a band-pass filter 43 connected to the circuit 42 to remove noises of the waveform signal in a predetermined range, a sine wave oscillating circuit 44 connected to the coils 41a to acquire a sine wave for excitation, a phase circuit 45 connected to the circuit 44 to shift a phase of the sine wave and an edge-trigger circuit 46 connected to the circuit 45 to convert the sine wave into a rectangular wave.

It is preferable that, upon setting or adjustment and with no magnetic particles being detected, the phase circuit 45 shifts the phase by 10°-170°, preferably by 45°-135°, and further preferably by about 90°. The phase circuit 45 may be alternatively positioned between the band-pass filter 43 and a signal processor 47 to shift not the phase of the reference signal, but the phases of the detection or corrective detection signal of the magnetic particles.

The signal processing unit 35 further comprises the signal processor 47 connected to the band-pass filter 43 and to the edge-trigger circuit 46, a low-pass filter 48 connected to the processor 47 to convert the output signal into a DC voltage signal, an amplifier 49 connected to the filter 48 to amplify the DC voltage signal, an AC signal transmitting circuit 50 connected to the amplifier 49 to transmit only an amount varied of the DC voltage signal by the guided lead-out/lead-in of the detection fluid and an amplifier 51 connected to the circuit 50. The signal processor 47 is preferably a lock-in amplifier; however, it may be any, provided that it can measure variation in phase difference.

The amplifier 51 of the signal processing unit 35 is connected to the concentration measuring unit 36 shown in Fig. 6 or 7 to convert the signal into the concentration of the magnetic particles.

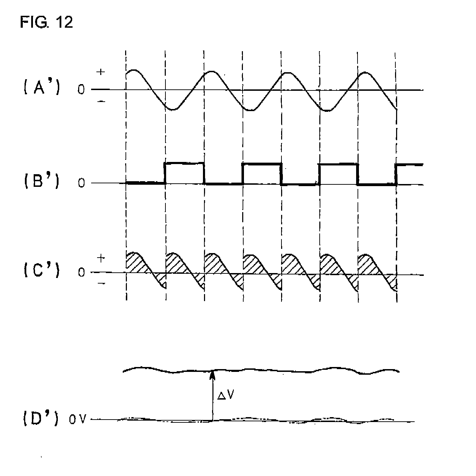

The concentration measuring unit 36 of the magnetic particle measuring part 2 is connected to the control part 3 for processing of the concentration of the magnetic particles. The control part 3 converts the concentration of the magnetic particles measured by the magnetic particle measuring part 2 into the concentration of hard particles in the oil S by contrast with a calibration line acquired in advance (see Fig. 12) to thereby detect and display the concentration of the hard particles in the oil S. The processes of the control part 3 may be executed manually, the manner of executing the processes being not especially limited.

An operation of the embodiment of the hard particle concentration detecting method according to the invention will be described.

When the fuel or other oil S which may include hard particles is to be tested, firstly a small amount of oil S (sample) for testing is sampled as shown in Fig. 1 from an inlet or the like of the engine in a fuel system (step S1). The fuel or other oil S to be tested is not limited to bunker C or other heavy oil and may be other type of oil S such as gasoline, kerosene or light oil, provided that it may include hard particles. The use of the oil S is not limited to supply to a driving engine of a ship or the like and may be supply to various kinds of driving engines and devices of, for example, a turbine plant. Water or a water solution may be used instead of an oil; the liquid is not especially limited, provided that it may include hard particles. In addition, when water or a water solution is to be tested, the embodiment may be used for detection of particle-like impurities mixed in circulation water of a water circulation compressor or the like, may be used for a water quality inspection of operating water of a hydraulic machine and may be used for water quality management of treated water in a water treatment facility. In addition, hard particles are non-conductive and non-magnetic particles included in a liquid such as the oil S or water and capable of wearing the magnetic member 12 and are not limited to alumina, silica or carbon particles.

Then, the small amount of oil S (sample) sampled is set in the magnetic particle measuring part 2 to measure a concentration (X) of magnetic particles originally included in the oil S (step S2). The processes by the magnetic particle measuring part 2 will be described hereinafter; alternatively, the concentration (X) of the magnetic particles may be measured using other devices. The processes from the sampling of the oil S to setting thereof in the magnetic particle measuring part 2 may be continuously conducted with no manpower through a supplying device such as an oil supply flow path.

After the concentration (X) of the magnetic particles originally included in the oil S is measured, the oil S (sample) is fed into the container 13 which is then set in the magnetic particle producing part 1 for preparation (step S3). More specifically, a spout opening of the container 13 with the oil S therein is fitted over and fixed to the protrusion 14 of the holder 6 such that the magnetic and counterpart members 12 and 11 together with the rod 7 and the like are immersed in the oil S. In any of the other configurations of the magnetic particle producing part 1 shown in Fig. 5, its magnetic and counterpart members are similarly immersed in the oil S. The transfer of the oil S from the magnetic particle measuring part 2 to the magnetic particle producing part 1 may be executed manually or automatically through a transferring device such as a flow path or an on-off valve.

Then, the magnetic particle producing part 1 is driven for a specific time period to produce magnetic particles such as iron powder in the oil S (step S4). More specifically, the drive 5 is driven to rotate the rod 7 and thus the magnetic member 12 while the member 12 is pressed against the counterpart member 11, so that the magnetic member 12 is worn by hard particles entering between the members 12 and 11 to produce magnetic particles due to the wearing of the member 12. In any of the other configurations of the magnetic particle producing part 1 shown in Fig. 5, the magnetic member 12 is similarly worn to produce the magnetic particles such as iron powder. In this step, a viscosity of the oil S is maintained constant so that, when the pressing contact pressure between the members 12 and 11 is kept properly, the magnetic particles (iron powder) are produced only by the hard particles of not less than a specific size. The hard particles of a size smaller than the specific size only pass through a gap between the members 12 and 11 and produce no magnetic particles (iron powder).

After the magnetic particles are produced in the oil S by the magnetic particle producing part 1, the oil S is set from the magnetic particle producing part 1 to the magnetic particle measuring part 2 for the next process. The transfer of the oil S from the magnetic particle producing part 1 to the magnetic particle measuring part 2 may be executed manually or automatically through a transferring device such as a flow path or an on-off valve,

Then, a concentration (Y) of the magnetic particles is measured by the magnetic particle measuring part 2 (step S5). For measurement of the concentration (Y) of the magnetic particles, the piston 39 of the fluid lead-out/lead-in guide unit 33 is continuously reciprocated to alternately and continuously repeat measuring processes with the oil S being introduced into and discharged from the detector body 38. The AC signal transmitting circuit 50 or the like detects a differential signal from output values for the concentration of the magnetic body and for comparison, executes a moving averaging process and acquires an average value of the concentration of the magnetic particles through the concentration measuring unit 36.

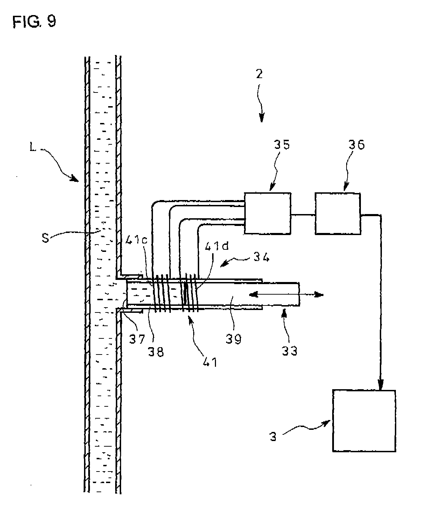

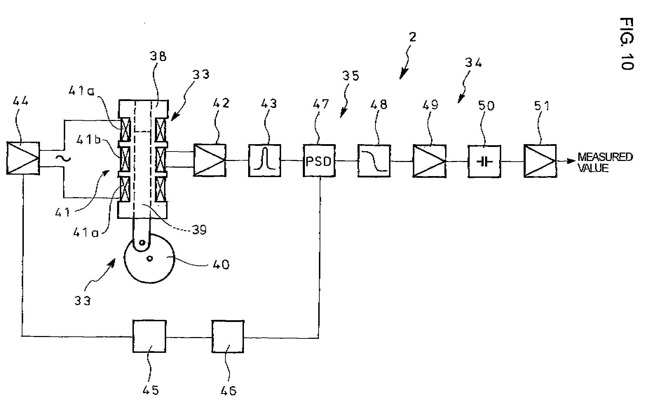

The process of measuring the concentration of the magnetic particles will be described in detail. When the oil S is discharged from the detector body 38, the corrective detection signal is acquired ((A) in Fig. 9) from the detector body 38 through the detection coil 41b, the amplifier circuit 42 and the band-pass filter 43 while the exciting coils 41a, the sine wave oscillating circuit 44, the phase circuit 45 and the edge-trigger circuit 46 are used to prepare, through a phase shift by a predetermined angle, a rectangular-wave reference signal which is at a same frequency as that of the exciting voltage and which generates a specific phase difference relative to the exciting voltage ((B) in Fig. 9 with a phase shift of about 90°). The signal processor 47 executes noise removal for the corrective detection signal together with the reference signal, and detects the phase difference between the corrective detection signal and the reference signal. The low-pass filter 48 converts the phase difference into a smooth DC voltage signal as the output value for the comparison ((D) in Fig. 9) which is input through the amplifier 49 into the AC signal transmitting circuit 50. On the other hand, when the oil S is introduced into the detector body 38, a detection signal of the magnetic body is acquired ((A') in Fig. 10) from the oil S through the detection coil 41b, the amplifier circuit 42 and the band-pass filter 43 while the exciting coils 41a, the sine wave oscillating circuit 44, the phase circuit 45 and the edge-trigger circuit 46 are used to prepare, through a phase shift by a predetermined angle, a rectangular-wave reference signal which is at a same frequency as that of the exciting voltage and which generates a specific phase difference relative to the exciting voltage ((B') in Fig. 10 with a phase shift of about 90°). The signal processor 47 executes noise removal for the detection signal together with the reference signal, and detects the phase difference between the detection signal of the magnetic body and the reference signal. The low-pass filter 48 converts the phase difference into a smooth DC voltage signal as the output value for the concentration of the magnetic body ((D') of Fig. 10) which is input through the amplifier 49 into the AC signal transmitting circuit 50. To correct the output value for the concentration of the magnetic particles, the AC signal transmitting circuit 50 acquires a difference ΔV from the output values for the concentration of the magnetic particles and for the comparison as shown in Fig. 10. The concentration measuring unit 36 converts the difference into the concentration of the magnetic particles based on a correlation (function process) acquired in advance between the difference and the concentration. (C) of Fig. 9 shows a state where the detection signal of the magnetic particles is inverted by the reference signal and conceptually shows that (D) of Fig. 9 is acquired by processing the area thereof by integration. (C') of Fig. 10 shows a state where the detection signal of the magnetic particles is inverted by the reference signal and conceptually shows that (D') of Fig. 10 is acquired by processing the area thereof by integration.

Results of experiments conducted by the inventors reveal that, when the oil S including magnetic particles (iron powder) in ppm was measured using the embodiment, an output (concentration) was increased simultaneously with inputting of the oil S and was decreased in association with discharge of the oil S. It was evident that the reaction of the embodiment against the magnetic particles was clear and quick and the concentration of the magnetic particles was able to be precisely measured.

After the concentration of the magnetic particles is measured by the magnetic particle measuring part 2, the concentration (X) of the magnetic particles originally included in the oil S is subtracted from the concentration (Y) of the magnetic particles acquired after the processing by the magnetic particle producing part 1 (the concentration (Y) of the magnetic particles - the concentration (X) of the magnetic particles) to thereby calculate the concentration (Z) of the magnetic particles actually produced by the magnetic particle producing part 1 (step S6).

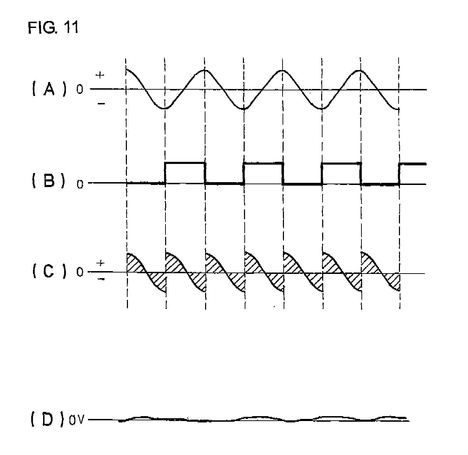

Testing of a relationship between the driving time period (grinding time period) of the magnetic particle producing part 1 and the concentration of the magnetic particles (magnetic powder Fe) revealed that the concentration of the magnetic particles in the oil S linearly increased with elapse of the grinding time period as shown in Fig. 11. With the concentration of the hard particles originally included in the oil S being varied (cases of α ppm, about α/2 ppm and no inclusion are shown in Fig. 11), it turned out that the concentrations of the hard particles and of the magnetic particles produced were in a proportional relationship. Based on this, when the concentrations of the hard particles and of the magnetic particles (magnetic powder) are plotted under the condition that the driving time period (grinding time period) of the magnetic particle producing part 1 is set to be a specific time period, a calibration line was drawn which represented the correlation between the concentrations of the magnetic particles and of the hard particles in the oil S as shown in Fig. 12. The calibration line is applied to the following processes,

After the concentration (Z) of the magnetic particles actually produced is calculated, the concentration of the magnetic particles is converted into the concentration of the hard particles in the oil S based on the calibration line (step S7, from A to B in Fig. 12). The conversion into the concentration of the hard particles may be processed after the calibration line is registered in advance in the control part 3 or may be processed manually.

The concentration of the hard particles is displayed on a displaying unit of the control part 3 or the like (step S8) to thereby detect and quickly and quantitatively grasp the hard particles in the oil S. Thus, the concentration of alumina, silica or other hard particles is determined on site during supply of fuel or other oil S to a driving engine of a ship or the like and any adverse effect to the driving engine caused by the hard particles is prevented in advance.

Thus, according to the embodiment of the hard particle concentration detecting method of the invention, the magnetic particles are produced by wearing the magnetic member 12 due to the presence of the hard particles in the oil S; the concentration of the magnetic particles produced in the oil S is measured; the concentration of the magnetic particles is converted into the concentration of the hard particles in the oil S based on the calibration line to detect the concentration of the hard particles included in the oil S. Thus, a specific number of days are not necessary to detect the concentration of the hard particles unlike the conventional measuring method, and the hard particles in the oil S can be quickly and quantitatively grasped. Therefore, the states can be prevented where untested fuel is used and where a large amount of hard particles are suddenly supplied to a driving engine. Thereby, any adverse effect on the driving engine can be suppressed. The concentration of the hard particles is indirectly detected using the magnetic particles such as iron powder produced by the wearing of the magnetic member 12, so that any operation and any process for direct detection of the hard particles by physically or chemically treating or processing the oil S itself are not required and favorably the hard particles in the oil S can be grasped quickly and quantitatively.

In the embodiment of the hard particle concentration detecting method of the invention, movement of at least one of the magnetic and counterpart members 12 and 11 to the other while pressed to the other and with the hard particles in the oil S being between the members 12 and 11 causes the magnetic member 12 to be properly worn out due to the presence of the hard particles in the oil S to thereby produce the magnetic particles. Thus, the concentration of the hard particles in the oil S can be easily detected to more favorably grasp the hard particles in the oil S.

When an invading path such as the groove 12a is arranged for at least one of the magnetic and counterpart members 12 and 11 for facilitated entering of the sample oil into the gap, it promotes the production and discharge of the magnetic particles (iron powder), whereby the magnetic particles are favorably produced. Thus, the concentration of the hard particles in the oil S can be easily detected and the hard particles in the oil S can be more favorably grasped.

The embodiment can be applied not only to oil but also to water and a water solution. Therefore, the embodiment is highly versatile and can easily detect the concentration of the hard particles from water or a water solution.

It is to be understood that a hard particle concentration detecting method of the invention is not limited to the above illustrated embodiment and that various changes and modifications may be made without departing from the scope of the invention.

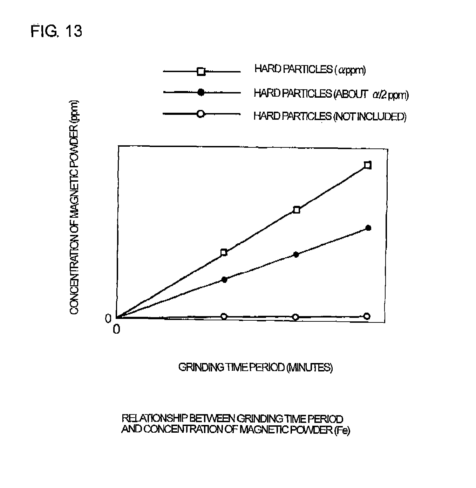

An embodiment of a particle concentration detecting method and a device therefor of the invention will be described with reference to Figs. 13 to 20. The embodiment will be described with respect to a case where a liquid flowing through a flow path is a fuel oil.

As shown in Fig. 13, a particle concentration detecting device 101 of the embodiment is arranged in a flow path L2 branched from a flow path L1 just before a prime mover C such that the concentration of particles (hard particles) can be measured without affecting the flow path L1 through which the fuel oil flows from a fuel service tank A via a buffer column B into the prime mover C. The flow path L2 finally discharges the fuel measured by the detecting device 101 to a sludge tank (not shown). With the construction of Fig. 13, the flow path L1 from the tank A to the column B has a fuel supply pump D, a bypass filter E, a fine filter F and the like and the flow path L1 from the column B to the prime mover C has a circulation pump G, a heater H, a filter I and a viscosity adjuster J. Return flow paths L3 and L4 are arranged from the buffer column B to the tank A and from the prime mover C to the buffer column B, respectively.

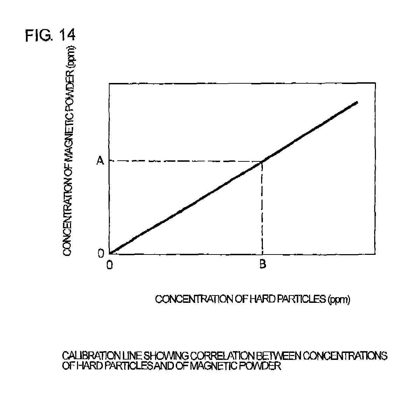

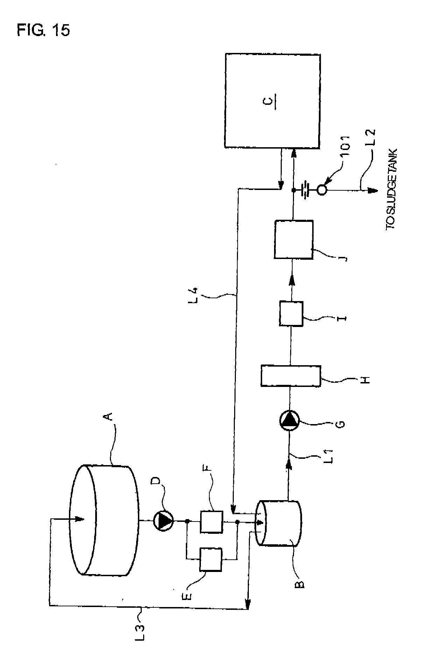

As shown in Figs. 14 and 15, the particle concentration detecting device 101 comprises a magnetic particle producing part 102 positioned at a turnaround portion of the flow path L2 to produce magnetic particles in the oil S, a magnetic particle measuring part 103 positioned in the flow path L2 like the producing part 102 to measure a concentration of the magnetic particles in the oil S, a control part 104 to process information from the measuring part 103, a temperature controlling unit 105 positioned in an inflow-side flow path L2a to control a temperature on the inflow side and a flow adjusting unit 106 with a gear pump 151 positioned in the inflow-side flow path L2a to feed the oil S at a specific flow rate.

As shown in Figs. 14 to 16a, the magnetic particle producing part 102 comprises a casing 107 into/from which the oil S in the flow path L2 flows, a motor or other drive 108 positioned above the casing 107 and having a rotating shaft 108a, a disc-like rotating pedestal 110 positioned above within the casing 107 through a connecting shaft 109 connected to the shaft 108a of the drive 108, a pedestal 112 positioned below within the casing 107 and biased upward from a bottom of the casing 107 by a spring or other resilient member 111, a plate-like magnetic member 114 fixed to a bottom face of the rotating pedestal 110 through a fixing pin or other fixing member 113, and a plate-like counterpart member 116 fixed to a top face of the pedestal 112 through a fixing pin or other fixing member 115 and surface-contacting a bottom face of the magnetic member 114. Sealing rings 117 are arranged between the casing 107 and the connecting shaft 109 and between the casing 107 and the pedestal 112 to prevent the oil S from leaking outside. The magnetic member 114 is made of an iron-based or other material having magnetism. The counterpart member 116 is made of a carbon steel or other material which is harder and less wearable than the magnetic member 114. The material of the magnetic member 114 is not limited to iron and may be any, provided that magnetic particles with a required particle diameter may be produced due to the wearing. The material of the counterpart member 116 may be different from or the same as that of the magnetic member 114, provided that the magnetic particles may be produced from the magnetic member 114. The magnetic and counterpart members 114 and 116 may be arranged with their positions exchanged.

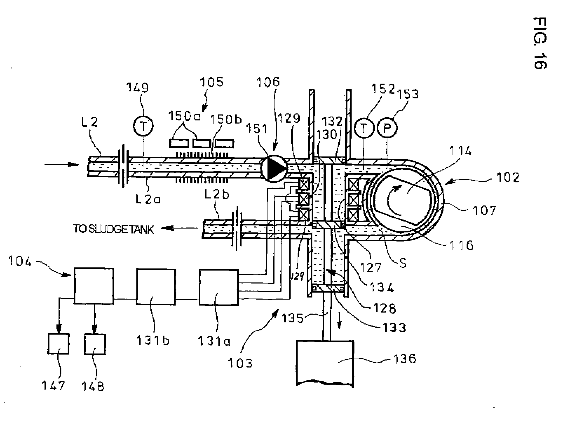

An alternative example of the magnetic particle producing part 102 may be employed as shown in Fig. 16b which comprises a casing 118 into/from which the oil S in the flow path L2 flows, a motor or other drive 119 positioned above the casing 118 and having a shaft 119a, a rod-like magnetic member 114a connected to the shaft 119a of the drive 119 and rotating in the casing 118 and a counterpart member 116a fitted over the magnetic member 114a and biased away from a side face of the casing 118 by springs or other resilient members 120. A sealing ring 121 is fitted between the casing 118 and the magnetic member 114a to prevent the oil S from leaking outside. The magnetic member 114a is made of an iron-based or other material having magnetism. The counterpart member 116a is made of carbon steel or other material which is harder and less wearable than the magnetic member 114a, The material of the magnetic member 114a is not limited to iron and may be any, provided that magnetic particles with a required particle diameter may be produced due to the wearing. The material of the counterpart member 116a may be different from or the same as that of the magnetic member 114a, provided that the magnetic particles may be produced from the magnetic member 114a. The magnetic and counterpart members 114a and 116a may be arranged with their positions exchanged.

A yet alternative example of the magnetic particle producing part 102 may be employed as shown in Fig. 16c, which comprises a casing 122 into/from which the oil S in the flow path L2 flows, a motor or other drive 123 positioned above the casing 122 and having a shaft 123a, a converter 124 which converts the rotation of the shaft 123a into a reciprocating motion using an eccentric pin or the like, a rod-like magnetic member 114b connected to the converter 124 and moving up and down in the casing 122 and a counterpart member 116b fitted over the magnetic member 114b and biased away from a side face of the casing 122 through springs or other resilient members 125. A sealing ring 126 is fitted between the casing 122 and the magnetic member 114b to prevent the oil S from leaking outside. The magnetic member 114b is made of an iron-based or other material having magnetism. The counterpart member 116b is made of carbon steel or other material which is harder and less wearable than the magnetic member 114b. The material of the magnetic member 114b is not limited to iron and may be any, provided that magnetic particles with a required particle diameter may be produced due to the wearing. The material of the counterpart member 116b may be different from or the same as that of the magnetic member 114b, provided that the magnetic particles may be produced from the magnetic member 114b. The magnetic and counterpart members 114b and 116b may be arranged with their positions exchanged.

The magnetic particle measuring part 103 comprises, as shown in Figs. 14, 15 and 17, a detector body 127 connected to the flow path L2 for the oil S, a movable partition 128 adapted to communicate the flow path L2 with an inside of the detector body 127 such that the oil S in the flow path L2 can be introduced into the detector body 127, two exciting coils 129 positioned outside of the detector body 127, an output coil 130 positioned outside of the detector body 127 and adjacent to the exciting coils 129, a signal processing unit 131a connected to the coils 129 and 130 and a concentration measuring unit 131b for conversion of a signal of the signal processing unit.

The detector body 127 is arranged communicably with an inflow-side flow path L2a to the magnetic particle producing part 102 and with an outflow-side flow path L2b from the producing part 102 for intercommunication therebetween. One and the other end of the detector body 127 extend outside of the flow paths L2a and L2b, respectively.

The movable partition 128 comprises an inflow-side piston body 132 which can move as a portion of an outside wall of the flow path L2a, an outflow-side piston body 133 which can move as a portion of an outside wall of the flow path L2b, an intermediate piston body 134 positioned between the inflow- and outflow-side piston bodies 132 and 133, a piston rod 135 having the piston bodies 132, 133 and 134 arranged thereon and a drive 136 such as a rotating body or a crank for reciprocating motion of the piston rod 135. Movement of the piston rod 135 in one direction (downward) as shown in Fig. 14 brings about switching into a state where the inflow-side flow path L2a is in communication with the inside of the detector body 127 due to the inflow-side and intermediate piston bodies 132 and 139, so that the oil S can flow through the flow path L2a into the detector body 127. On the other hand, movement of the piston rod 135 in the other direction (upward) as shown in Fig. 15 brings about switching into a state where the outflow-side flow path L2b is in communication with the inside of the detector body 127 due to the outflow-side and intermediate piston bodies 133 and 134, so that the oil S can flow through the flow path L2b into the detector body 127. Furthermore, the oil S introduced into the detector body 127 on the flow path L2a side is pushed out from the inside of the detector body 127 into the inflow-side flow path L2a and the one outside by the detector bodies 132 and 134 and is guided downstream by newly flowing oil S when the piston rod 135 is moved in the other direction (upward); the oil S introduced into the detector body 127 on the flow path L2b side is pushed out from the inside of the detector body 127 into the outflow-side flow path L2b and the other outside by the detector bodies 133 and 134 and is guided downstream by newly flowing oil S when the piston rod 135 is moved in the one direction (downward). During the reciprocating movement of the piston rod 135, the intermediate piston body 134 is caused to move from an inner wall of the flow path L2a to an inner wall of the flow path L2b or from the inner wall of the flow path L2b to the inner wall of the flow path L2a so as to cause the oil S to flow between the two exciting coils 129 and the output coil 130.

The exciting coils 129 are two coils wound in opposite directions to each other, connected in series and arranged at a predetermined spacing from each other. The output coil 130 is arranged between and adjacent to the exciting coils 129. The output coil 130 is adapted to generate an output signal of an AC voltage (exciting voltage) when the AC voltage is applied to the exciting coils 129. The exciting and output coils 129 and 130 are adjusted to have substantially uniform mutual inductance by adjusting the wound number and distance of the coils. The exciting and output coils 129 and 130 have no limitation in their numbers; one exciting coil 129 and one output coil 130 may be used.

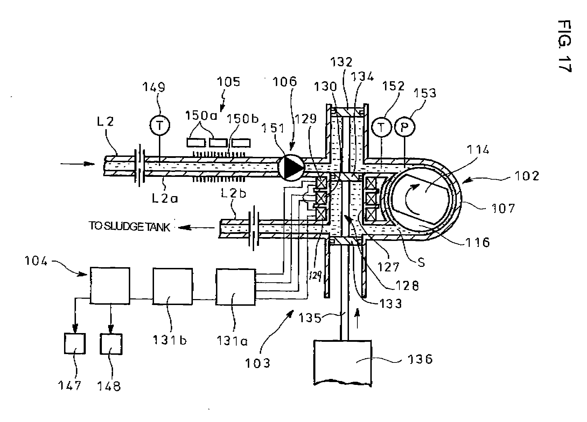

As shown in Fig. 17, in order to acquire detection or corrective detection signal of the magnetic particles from the output signal of the output coil 130, the signal processing unit 131a comprises an amplifier circuit 137 connected to the coil 130 to amplify a feeble waveform signal, a band-pass filter 138 connected to the circuit 137 to remove noises of the waveform signal in a predetermined range, a sine wave oscillating circuit 139 connected to the coils 129 to acquire a sine wave for excitation, a phase circuit 140 connected to the circuit 139 to shift the phase of the sine wave and an edge-trigger circuit 141 connected to the circuit 140 to convert the sine wave into a rectangular wave.

It is preferable that, upon settling or adjustment and with no magnetic particles being detected, the phase circuit 140 shifts the phase by 10°-170°, preferably by 45°-135°, and further preferably by about 90°. The phase circuit 140 may be alternatively positioned between the band-pass filter 138 and a signal processor 142 to shift not the phase of the reference signal, but the phases of the detection or corrective detection signal of the magnetic particles.

The signal processing unit 131a further comprises the signal processor 142 connected to the band-pass filter 138 and to the edge-trigger circuit 141, a low-pass filter 143 connected to the processor 142 to convert the output signal into a DC voltage signal, an amplifier 144 connected to the filter 143 to amplify the DC voltage signal, an AC signal transmitting circuit 145 connected to the amplifier 144 to transmit only an amount varied of the DC voltage signal by the guided lead-out/lead-in of the oil S and an amplifier 146 connected to the circuit 145. The signal processor 142 is preferably a lock-in amplifier; however, it may be any, provided that it can measure variation in phase difference.

The amplifier 146 of the signal processing unit 131a (see Fig. 17) is connected to the concentration measuring unit 131b shown in Figs. 14 and 15 to convert the signal into the concentration (concentration signal) of the magnetic particles based on a measured value.

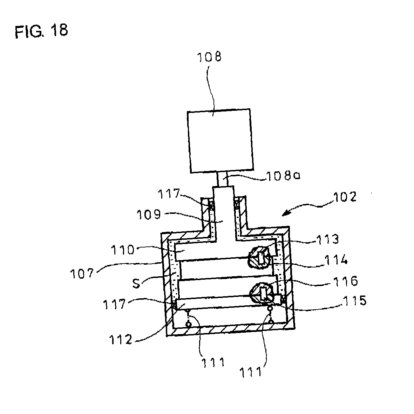

As shown in Fig. 18, the magnetic particle measuring part 103 may be arranged as succeeding and preceding magnetic particle measuring parts 103b and 103a in the outflow- and inflow-side flow paths L2b and L2a, respectively. In this case, the succeeding magnetic particle measuring part 103b comprises a detector body 127b connected only to the inflow-side flow path L2 and a piston 128b which guides out/in the oil S from the flow path L2 to the detector body 127b and has the same configuration as the configuration shown in Fig. 14 except the above components. The preceding magnetic particle measuring part 103a comprises a detector body 127a connected only to the inflow-side flow path L2 and a piston 128a which guides out/in the oil S from the flow path L2 to the detector body 127, and is adapted to send signals from the exciting coils 129 and the output coil 130 to the signal processor of the succeeding magnetic particle measuring unit 103b. A reference symbol α in the upper portion of Fig. 18 indicates connection to another reference symbol a in the lower portion thereof.

The concentration measuring unit 131b of the magnetic particle measuring part 103 is connected to the control part 104 shown in Figs. 14 and 15. The control part 104 is adapted to convert the concentration (concentration signal) of the magnetic particles measured by the measuring part 103 into the concentration of particles in the oil S by contrast with a calibration line (see Fig. 12) representing the correlation between the concentrations of the magnetic particles and of particles in the oil S. The control part 104 includes a displaying unit 147 to display the concentration of the particles and a warning unit L48 to output a warning sound, indication or the like.

Testing of a relationship between the driving time period (grinding time period) of the magnetic particle producing part 102 and the concentration of the magnetic particles (magnetic powder Fe) revealed that the concentration of the magnetic particles in the oil S was linearly increased with elapse of the grinding time period (see Fig. 11). With the concentration of the particles originally included in the oil S being varied to α ppm, about α/2 ppm and no particle included (zero ppm) as shown in Fig. 11, it turned out that the concent=ations of the particles and of the magnetic particles produced were similarly in a proportional relationship. Thus, the calibration line of the control part 104 is produced by comparing the concentrations of the particles (hard particles) and of the magnetic particles (magnetic powder) under the condition that the driving time period (grinding time period) of the magnetic particle producing part 102 is made constant.

The temperature controlling unit 105 comprises a thermometer 149 positioned upstream of the inflow-side flow path L2a and cooling fans and fins 150a and 150b positioned between the thermometer 149 and the measuring part 103 so as to cool down the inflow-side flow path L2a. The flow adjusting unit 106 is provided by a gear pump 151 positioned between the temperature controlling unit 105 and the measuring part 103. A thermometer 152 and a pressure gauge 153 are arranged in the inflow-side flow path L2a between the measuring and producing parts 103 and 102.

An operation of the embodiment of the particle concentration detecting method and the device therefor of the invention will be described.

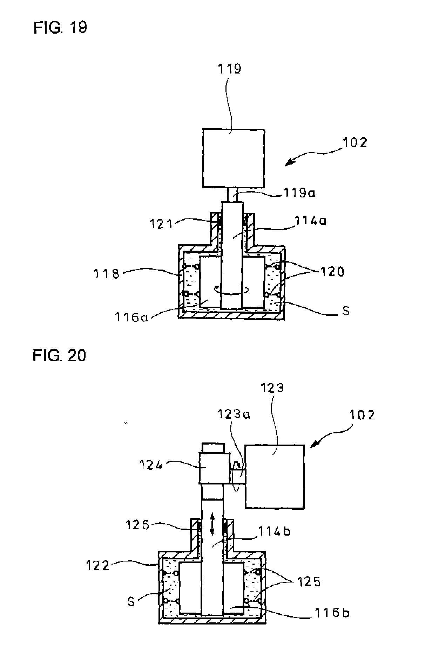

When the fuel or other oil (sample) S which may include particles is to be tested, the oil S is caused to flow into the particle concentration detecting device 101 in the flow path L2 branching just before the prime mover C (step S11 in Fig. 19). The fuel or other oil S to be tested is not limited to bunker C or other heavy oil and may be other type of oil such as gasoline, kerosene or light oil, provided that it may include particles. The use of the oil S is not limited to supply to the driving motor C of a ship or the like and may be supply to various kinds of driving engines and devices of, for example, a turbine plant. Water or a water solution may be used instead of the oil S; the liquid is not especially limited, provided that it may include particles. In addition, when water or a water solution is to be tested, the embodiment may be used for detection of particle-like impurities mixed in circulation water of a water circulation compressor or the like, may be used for a water quality inspection of operating water of a hydraulic machine and may be used for water quality management of treated water in a water treatment facility. In addition, particles are non-conductive and non-magnetic hard particles included in a liquid such as the oil S or water and capable of wearing the magnetic member 114 and are not limited to alumina, silica or carbon particles.

In the detecting device 101, firstly the temperature of the oil (sample) S is measured by the thermometer 149 of the temperature controlling unit 105. On the basis of the measured temperature of the oil S, the inflow-side cooling-down is conducted as needs demand by the cooling fan 150a or the like to thereby control the temperature of the oil S (step S12). In the case of the fuel oil S flowing from the buffer column B to the driving motor C, the temperature of the oil S may be one hundred and several ten degrees; it is, therefore, preferable that the oil S is cooled to 40 to 60 degrees so as not to affect on the measurement by the magnetic particle measuring part 103 and the durability of the magnetic particle measuring and producing parts 103 and 102.

The flow adjusting unit 106 or gear pump 151 adjusts a flow rate of the oil (specimen) S to be constant and decreases the pressure (step S13) to thereby stabilize the measurement and the processing by the magnetic particle measuring and producing parts 103 and 102, respectively.

Then, during passing of the oil (sample) S through the magnetic particle measuring part 103 in the inflow-or upstream-side flow path L2 (step S14), the piston rot 135 is moved in one direction (downward in Fig. 14) for switching into a state where the inflow-side flow path L2a is communicated with the inside of the detector body 127 due to the inflow-side and intermediate piston bodies 132 and 134, so that the oil S flowing from the flow path L2a into the magnetic particle producing part 102 is introduced into the detector body 127 for measurement of the concentration signal of the magnetic particles originally included in the oil S.

Then, during passing of the oil (sample) S through the magnetic particle producing part 102 (step S15), the drive 108 is driven to rotate the magnetic member 114 through the connecting shaft 109, the rotating pedestal 110 and the like while the magnetic member 114 is pressed to the counterpart member 116, so that the magnetic member 114 is abrasively worn by particles entering between the magnetic and counterpart members 114 and 116 to thereby produce the magnetic particles in the oil S. In the other configuration of the magnetic particle producing part 102 shown in Fig. 16b or 16c, similarly, the magnetic member 114a or 114b is abrasively worn to produce the magnetic particles in the oil S. The viscosity of the oil S is maintained constant so that, when the pressing contact pressure between the members 114 and 116 is kept at a proper pressure, the magnetic particles (iron powder) are produced only by the particles (hard particles) not less than a specific size. The particles less than the specific size only pass through the gap between the members 114 and 116 and produce no magnetic particles. For the magnetic member 114a or 114b and the counterpart member 116a or 116b, similarly, the magnetic particles (iron powder) are produced only by particles not less than a specific size and the particles less than the specific size produce no magnetic particles.

Then, during passing of the oil (sample) S through the magnetic particle measuring part 103 in the outflow- or downstream-side flow path L2 (step S16), the piston rod 135 is moved in the other direction (upward in Fig. 15) for switching into a state where the flow path L2b is in communication with the inside of the detector body 127 due to the outflow-side and intermediate piston bodies 133 and 134, so that the oil S flowing from the magnetic particle producing part 102 to the outflow-side flow path L2b is introduced into the detector body 127 for measurement of the concentration signal of the magnetic particles on the outflow side. After detection of the concentration of the magnetic particles in the outflow-side flow path L2b, the oil S is returned to the flow path L2b through the movement of the movable partition 128 of the magnetic particle measuring part 103 and is discharged from the flow path L2b to the sludge tank (see Fig. 13) through an orifice (not shown) or the like (step S17).

For measurement of the concentration of the magnetic particles, the piston rod 135 of the movable partition 128 is continuously reciprocated to alternately execute the measurements in the state where the oil S in the inflow-side flow path L2a is introduced into the detector body 127 and in the state where the oil S in the outflow-side flow path L2b is introduced into the detector body 127. The concentration (concentration signal) P1 of the magnetic particle originally included in the oil S is subtracted from the concentration (concentration signal) P2 of the outflow side magnetic particles as shown in Fig. 20 to thereby calculate the concentration (concentration signal) ΔS of the magnetic particles produced by the magnetic particle producing part 102. In Fig. 20, positions of P1 and P2 are positions at which the movable partition 128 of the magnetic particle measuring part 103 introduces the oil S from the inflow- and outflow-side flow paths L2a and L2b, respectively.

The process of measuring the concentration of the magnetic particles by the signal processing unit 131a and the concentration measuring unit 131b will be described in detail (see Figs. 9 and 10). When the oil S is introduced from the inflow-side flow path L2a into the detector body 127, the signal processing unit 131a acquires the corrective detection signal ((A) in Fig. 9)from the detector body 127 through the output coil 130, the amplifier circuit 137 and the band-pass filter 138 while the exciting coils 129, the sine wave oscillating circuit 139, the phase circuit 140 and the edge-trigger circuit 141 are used to prepare, through a phase shift by a predetermined angle, a rectangular-wave reference signal which is at a same frequency as that of the exciting voltage and which generates a specific phase difference relative to the exciting voltage ((B) in Fig. 9 with a phase shift of about 90°), The signal processor 142 executes noise removal for the corrective detection signal together with the reference signal, and detects the phase difference between the corrective detection signal and the reference signal. The low-pass filter 143 converts the phase difference into a smooth DC voltage signal as the output value (the concentration of the magnetic particles originally included in the flow path L2) for the comparison ((D) in Fig. 9) which is input through the amplifier 144 into the AC signal transmitting circuit 145. On the other hand, when the oil S is introduced from the outflow-side flow path L2b into the detector body 127, a detection signal of the magnetic particles is acquired ((A') in Fig. 10) from the oil S through the output coil 130, the amplifier circuit 137 and the band-pass filter 138 while the exciting coils 129, the sine wave oscillating circuit 139, the phase circuit 140 and the edge-trigger circuit 141 are used to prepare, through a phase shift by a predetermined angle, a rectangular-wave reference signal which is at a same frequency as that of the exciting voltage and which generates a specific phase difference relative to the exciting voltage ((B') in Fig. 10 with a phase shift of about 90°). The signal processor 142 executes noise removal for the detection signal together with the reference signal, and detects the phase difference between the detection signal of the magnetic particles and the reference signal. The low-pass filter 143 converts the phase difference into a smooth DC voltage signal as the output value for the concentration of the magnetic particles ((D') in Fig. 10) which is input through the amplifier 144 into the AC signal transmitting circuit 145. To subtract the concentration of the magnetic particles originally included in the flow path L2, the AC signal transmitting circuit 145 acquires a difference ΔV from the output value for the concentration of then magnetic particles and the output value for the comparison as shown in Fig. 10; the measured value is transmitted through the amplifier 146 to the concentration measuring unit 131b which then converts the measured value of the difference into a concentration (concentration signal) ΔS of the magnetic particles based on the correlation (function process) acquired in advance between the difference and the concentration. (C) of Fig. 9 shows the state where the detection signal of the magnetic particles is inverted by the reference signal and conceptually shows that (D) of Fig. 9 is acquired by processing the area thereof by integration. (C') of Fig. 10 shows the state where the detection signal of the magnetic particles is inverted by the reference signal and conceptually shows that (D') of Fig. 10 is acquired by processing the area thereof by integration.

After the concentration ΔS of the magnetic particles is calculated, the control part 104 converts the concentration of the magnetic particles into the concentration of the particles in the oil S based on the calibration line and displays the concentration of the particles on the displaying unit 147. When the concentration of the particles exceeds a predetermined threshold value, the warning unit 148 outputs a warning sound, indication or the like. Alternatively, the concentration of the particles may be converted directly from the stage of the difference ΔV of the AC signal transmitting circuit 145 into the concentration of the particles through no processing by the concentration measuring unit 131b or may be processed according to any other procedure. The predetermined threshold value may be properly set based on, for example, a permissible amount of particles which may flow into the driving motor C.

Thus, when the fuel or other oil S is to be supplied to the driving motor C of a ship or the like, the concentration of the alumina, silica and other particles is monitored on site to preliminarily avoid any adverse effect on the driving engine caused by the particles.

In the case of the alternative example of the particle concentration detecting device 101 shown in Fig. 18, the preceding and succeeding magnetic particle measuring parts 103a and 103b in the inflow- and outflow-side flow paths L2a and L2b separately measure the concentration (concentration signal) of the magnetic particles. The concentration (concentration signal) of the magnetic particles originally included in the oil S is subtracted from the concentration (concentrations signal) of the magnetic particles on the outflow side to thereby calculate the concentration (concentration signal) of the magnetic particles produced by the magnetic particle producing part 102. The calculated concentration of the magnetic particles is converted by the control part 104 into the concentration of the particles in the oil S on the basis of the calibration line.

The process of measuring the concentration of the magnetic particles by the magnetic particle measuring part 103b shown in Fig. 18 will be described. The signal processing unit 131a acquires the difference ΔV of the AC signal transmitting circuit 145 by comparing the case where the oil S is discharged from the detector body 127 with the case where the oil S is introduced into the detector body 127, and the concentration measuring unit 131b acquires the concentration of the magnetic particles from the difference ΔV. In the case of the magnetic particle measuring part 103a, the concentration of the magnetic particles is acquired in the same manner as that of the magnetic particle measuring part 103b.

Thus, according to the embodiment of the particle concentration detecting method and the device therefor of the invention, the magnetic particles are produced by wearing the magnetic member 114 due to the presence of the particles in the oil S; the concentration of the magnetic particles produced in the oil S is measured; and the concentration of the magnetic particles is converted into the concentration of the particles in the oil S on the basis of the calibration line to thereby detect the concentration of the particles included in the oil S. Therefore, a specific number of days are not required to detect the concentration of the particles unlike the conventional measuring methods, and the particles in the oil S can be quantitatively grasped. At the same time, the magnetic particle producing and measuring parts 102 and 103 are arranged in the same flow path L2, so that the concentration of the particles in the liquid can be continuously grasped.

The continuous and quantitative grasp of the particles in the oil S can prevent the states where untested fuel is used and where a large amount of particles are suddenly supplied to a driving engine, so that any adverse effect on the driving engine can be suppressed. The concentration of the particles is indirectly detected using the magnetic particles such as iron powder produced by the wearing of the magnetic member 114, which eliminates the use of any operation and process for direct detection of the particles through physical or chemical processing of the oil S itself and favorably enables continuous and quantitative grasp of the particles in the oil S.

According to the embodiment of the particle concentration detecting method and the device therefor of the invention, the concentration of the magnetic particles originally included in the oil S is measured before the production of the magnetic particles by the magnetic particle producing part 102 and is subtracted from the concentration of the magnetic particles produced in the liquid by the producing part 102; and the subtraction result is converted into the concentration of the particles. By such measurement of the concentration of only the magnetic particles produced in the oil by the magnetic particle producing part 102, the particles in the oil S can be favorably grasped.

The detector body 127 of the magnetic particle measuring part 103 is arranged for communication with the inflow- and outflow-side flow paths L2a and L2b to and out of the magnetic particle producing part 102. The movable partition 128 of the magnetic particle measuring part 103 comprises the inflow-side piston body 132 arranged for the inflow-side flow path L2a, the outflow-side piston body 133 arranged for the outflow-side flow path L2b, the intermediate piston body 134 arranged between the inflow- and outflow-side piston bodies 132 and 133, and the reciprocating piston rod 135 having the piston bodies 132, 133 and 134 arranged thereon. The movement of the piston rod 135 in the one direction brings about switching into the state where the inflow-side flow path L2a is in communication with the inside of the detector body 127 due to the inflow-side and intermediate piston bodies 132 and 134 and the oil S flowing through the flow path L2a is introduced into the detector body 127. The movement of the piston rod 135 in the other direction brings about switching into the state where the outflow-side flow path L2b is in communication with the inside of the detector body 127 due to the outflow-side and intermediate piston bodies 133 and 134 and the oil S flowing through the flow path L2b is introduced from the flow path L2b into the detector body 127. Due to the above configuration, the single magnetic particle measuring part 103 can easily measure the concentration of the magnetic particles originally included in the liquid and properly measure the concentration of the magnetic particles by the magnetic particle producing part 102 to thereby favorably grasp the particles in the oil S.

It is to be understood that a particle concentration detecting method and a device therefor of the invention are not limited to the above illustrated embodiment and that various changes and modifications may be made without departing from the scope of the invention.

### Reference Signs List

- 1: magnetic particle producing part
- 2: magnetic particle measuring part
- 11: counterpart member
- 12: magnetic member
- 101: concentration detecting device
- 102: magnetic particle producing part
- 103: magnetic particle measuring part
- 103a: magnetic particle measuring part
- 103b: magnetic particle measuring part
- 104: control part
- 105: temperature controlling unit
- 106: flow adjusting unit
- 114: magnetic member
- 114a: magnetic member
- 114b: magnetic member
- 116: counterpart member
- 116a: counterpart member
- 116b: counterpart member
- 127: detector body
- 127a: detector body
- 127b: detector body
- 12B: movable partition
- 12Ba: movable partition
- 128b: movable partition
- 129: exciting coil
- 130: output coil
- 131a: signal processing unit
- 132: inflow-side piston body
- 133: outflow-side piston body
- 134: intermediate piston body
- 135: piston rod
- S: oil (liquid)

## Claims

1. A hard particle concentration detecting method comprising the steps of immersing magnetic and counterpart members (12),(11) in a liquid (S) which may include hard particles, moving at least one of the members while the member is pressed to the other member, wearing the magnetic member (12) by hard particles in the liquid (S) to produce magnetic particles in the sample liquid (S), measuring a concentration of the magnetic particles produced, and converting the measured concentration of the magnetic particles into a concentration of hard particles in the liquid (S) on the basis of a calibration line representing a correlation measured in advance between the concentrations of the magnetic particles and of the hard particles in the liquid (S) to thereby detect the concentration of the hard particles in the liquid (S).

2. The hard particle concentration detecting method as claimed in claim 1, therein said at least one of magnetic and counterpart members (12),(11) is moved while pressed to the other member and with the hard particles in the liquid (S) being between the members (12),(11).

3. A particle concentration detecting method providing a magnetic particle producing part positioned in a flow path of a liquid which may include particles and having magnetic and counterpart members arranged therein as well as a magnetic particle measuring part positioned in the flow path same as that for the magnetic particle producing part to measure a concentration of magnetic particles in the liquid, said method comprising the steps of, when a concentration of particles is to be measured,
moving at least one of the members while the member is pressed to the other member, wearing the magnetic member to produce magnetic particles in the liquid, measuring a concentration of the produced magnetic particles by the magnetic particle measuring part, converting the measured concentration of the magnetic particles into a concentration of particles in the liquid on the basis of a calibration line representing a correlation measured in advance between the concentrations of the magnetic particles and of the particles in the liquid, thereby detecting the concentration of the particles in the liquid.

4. The particle concentration detecting method as claimed in claim 3, wherein a concentration of magnetic particles originally included in the liquid is measured before the production of the magnetic particles by the magnetic particle producing part, said concentration of the magnetic particles originally included in the liquid being subtracted from the concentration of the magnetic particles produced in the liquid by the magnetic particle producing part, a subtracted result being converted into the concentration of the particles.

5. A particle concentration detecting device comprising
a magnetic particle producing part having magnetic and counterpart members arranged in a flow path of a liquid which may include particles for moving at least one of said members while the member is pressed to the other member and wearing the magnetic member to produce magnetic particles,
a magnetic particle measuring part positioned in the same flow path as that of the magnetic particle producing part for measuring the concentration of the magnetic particles in the liquid, and
a control part for converting the concentration of the magnetic particles measured by the magnetic particle measuring part into a concentration of the particles in the liquid on the basis of a calibration line representing a correlation measured in advance between concentrations of the magnetic particles and of the particles in the liquid, thereby detecting the concentration of the particles included in the liquid.

6. The particle concentration detecting device as claimed in claim 5, wherein a preceding magnetic particle measuring part is arranged upstream of the magnetic particle producing part to measure a concentration of the magnetic particles originally included in the liquid.

7. The particle concentration detecting device as claimed in claim 5 or 6, wherein the magnetic particle measuring part comprises a detector body connected to the flow path of the liquid, a movable partition for communication of the flow path with an inside of the detector body such that the liquid in the flow path may be introduced into the detector body, an exciting coil positioned external to the detector body, an output coil positioned external to the detector body for generating an exciting voltage by an AC current of the exciting coil and a signal processing unit for measuring a variation in phase difference between the exciting and output coils.

8. The particle concentration detecting device as claimed in claim 7, wherein the detector body of the magnetic particle measuring part is arranged communicably with an inflow-side flow path to the magnetic particle producing part and with an outflow-side flow path from the magnetic particle producing part,
the movable partition of the magnetic particle measuring part comprising an inflow-side piston body arranged for the inflow-side flow path, an outflow-side piston body arranged for the outflow-side flow path, an intermediate piston body arranged between the inflow-and outflow-side piston bodies and a piston rod having the inflow- and outflow-side and intermediate piston bodies arranged thereon,
whereby movement of the piston rod in one direction brings about switching into a state where the inflow-side flow path is in communication with the inside of the detector body due to the inflow-side and intermediate piston bodies and the liquid flowing through the inflow-side flow path is introduced into the detector body; and movement of the piston rod in the other direction brings about switching into a state where the outflow-side flow path is in communication with the inside of the detector body due to the outflow-side and intermediate piston bodies and the liquid flowing through the outflow-side flow path is introduced into the detector body.

9. The particle concentration detecting device as claimed in claim 5, wherein the inflow-side flow path is provided with a temperature controlling unit for controlling a temperature on the inflow side and a flow adjusting unit for feeding the liquid at a constant flow rate.
